# EUROPEAN PATENT SPECIFICATION

(11) **EP 1 910 040 B1**
(45) Date of publication and mention of the grant of the patent: **17.12.2008**
(21) Application number: 06755719.9
(22) Date of filing: 06.07.2006
(51) Int. Cl.: B25J 9/16, A61B 19/00

(54) **A ROBOT AND A METHOD OF REGISTERING A ROBOT**
ROBOTER UND VERFAHREN ZUR REGISTRIERUNG EINES ROBOTERS
ROBOT ET PROCÉDÉ D'ENREGISTREMENT D'UN ROBOT

(30) Priority: 06.07.2005 GB 0513876
(43) Date of publication of application: 16.04.2008
(73) Proprietor: Prosurgics Limited, High Wycombe Buckinghamshire HP10 9QR (GB)
(72) Inventor: FINLAY, Patrick Armstrong, Bucks HP9 1AE (GB)
(74) Representative: Beattie, Alex Thomas Stewart
(86) International application number: PCT/GB2006/002501
(87) International publication number: WO 2007/003949

(56) References cited:
- WO-A-20/05032390
- US-A- 6 167 292
- US-B1- 6 430 434

## Description

### Description of Invention

**THE PRESENT INVENTION** relates to a robot and relates to a method of registering a robot.

When a robot is to act on a work piece it is necessary for the precise orientation and position of the work piece to be determined within the spatial frame of reference of the robot, so that the robot can operate accurately on the work piece, performing desired operations at precisely predetermined points on the work piece.

In many situations where robots are used, the robot is programmed to operate on a work piece of a precisely known size and shape, which is presented to the robot in a predetermined position relative to the robot. An example of such a situation is where a robot operates on a motor vehicle assembly line, where each work piece is of a precisely known size and shape, and is located in a precisely defined work station. In such a situation the robot can be preprogrammed to carry out a sequence of moves which are appropriate for the task that the robot has to perform.

However, if the workpiece is not in a predetermined position before the robot can perform the operations it is necessary for the precise position and orientation of the work piece to be determined within the frame of reference of the robot before the robot can perform any moves relative to the work piece. There are also situations where a robot has to perform tasks on a work piece where the size and shape and other characteristics of the work piece are known approximately, but the precise details differ from specimen to specimen. Examples may include hand made items, and items made of semirigid or deformable material, but a particular example is living tissue, for example, where the living tissue forms part of a patient, and where the robot is used in an operating theatre to hold or guide specific instruments or other tools used by a surgeon.

Where a robot is used in an operating theatre is not uncommon for the task of the robot to involve the steps of penetrating the "work piece" or a patient in order to access a particular internal target or pathway. In many cases the internal target or pathway is totally invisible from the surface of the work piece or patient, especially in the situation of a robot acting on a human patient in an operating theatre. It is, however, essential that the robot should access the internal target or pathway accurately.

A convenient method which has been used for specifying appropriate co-ordinates and instructions to the robot for these types of tasks involves the use of an image guided technique. In utilising this technique, an image is acquired of the "work piece" (which may be just one part of a patient, for example the head of a patient) by using X-rays, magnetic resonance imaging, ultra-sound imaging or some other corresponding technique. The imaging technique that is utilised is chosen so that the internal target or pathway is revealed or can be determined.

Such a method of registering a patent relative to a surgical robot and the corresponding robot are known from document US-A- 6167292.

So that there is a specific frame of reference which can be used to determine the absolute position of the internal target or pathway, a series of "markers" which will be visible within the generated image are mounted on the relevant part of the patient. The markers may be small metallic markers mounted on the head of the patient, for example.

An image of the relevant part of the patient is thus generated, and the image can be computer processed and displayed in a form that is convenient for a human operator. Depending upon the preference of the operator, and the nature of the internal target or pathway, the image may be presented as a series of "slices" through the work piece, or as three orthogonal views through a designated point, or, alternatively, as a three-dimensional reconstruction. There are many types of imaging processing algorithms available for this purpose.

Using an appropriate pointing device, such as a mouse, a human operator can now specify on the computer processed image of the relevant part of the patient where a target is located. The target may, for example, be a tumour. The operator may also indicate an appropriate approach path for the robot to reach the target. The target or required approach path are effectively defined relative to a frame of reference, which constitutes a set of three-dimensional spatial co-ordinates, but the positions of the markers are also defined with reference to the same frame of reference or the same spatial co-ordinates.

The co-ordinates of the key points of the desired approach path, and also the target itself are readily determinable from the pixel or voxel which the operator has specified with the pointing device.

Once the target and pathway have been defined, a series of instructions can be generated which can be utilised by the control arrangement of a robot so that the robot effects the appropriate moves to cause an instrument or end effector carried by the robot to follow the desired path to the target.

However, the instructions refer to the frame of reference of the images of the relevant part of the patient, and a robot will have its own "internal" frame of reference.

Thus, before the robot can be utilised to carry out the instructions provided by the robot controller, a "registration" process must be performed to "register" or correlate the internal frame of reference of the robot with the frame of reference of the images of the relevant part of the patient. In this way it can be ensured that when the robot carries out the instructions, the instrument or end effector carried out by the robot actually follows the correct path and effects the appropriate movements.

It has been proposed to provide a robot and to register the position of the robot relative to an object such as part of the patient (see WO 99/42257), by using a camera mounted on part of the robot which can acquire images of the markers used when initially preparing the computer processed image of the relevant part of the patient. Consequently, the camera on the robot can acquire images of the markers, and can determine the precise position of those markers within the internal frame of reference or internal spatial co-ordinates of the robot. However, because the position of the markers relative to the frame of reference or spatial co-ordinates used when the initial image was acquired are known, the frame of reference of the patient can be correlated with the frame of reference of the robot, and thus the precise co-ordinates of the target or path, as defined in the frame of reference of the images of the patient can easily be "translated" into the corresponding co-ordinates in the frame of reference of the robot, thus enabling the robot to follow the appropriate series of instructions.

When an arrangement of this type is utilised, it is conventional for the part of the patient to be operated on to be clamped firmly in position, and for the robot then to be calibrated, by effectively correlating the internal frame of reference of the robot with the frame of reference of the images of relevant part of the patient. Because the main part of the robot and the relevant part of the patient are both fixed firmly in position, the robot can then follow the prepared set of instructions, moving the instrument or end effector accurately in the predetermined manner.

However, should the relevant part of the patient move, then the robot may no longer be used, because there is no correlation between the frame of reference of the relevant part of the patient and the frame of reference of the robot. Correlation cannot be effected again at this stage because, typically, the relevant part of the patient has been draped with sterile drapes, rendering the markers invisible to the camera carried by the robot.

The present invention seeks to provide an improved robot and an improved method.

According to one aspect of this invention there is provided a robot, the robot being provided with a controllable arm to carry an instrument or tool, the robot being provided with a visual image acquisition device to obtain visual images of a work piece, including images of markers and an indicator present on the work piece, the robot incorporating a processor to process the images, the processor being configured to determine the position of the markers within a spatial frame of reference of the robot to determine the position of the work piece in the spatial frame of reference of the robot and to control the robot to effect predetermined movements of an instrument or tool carried by the arm relative to the work piece, the processor being further configured to determine the position of said indicator and to respond to movement of the said indicator within the spatial frame of reference of the robot when the markers are concealed to determine the new position of the indicator and thus the new position of the work piece and subsequently to control the robot to continue effecting the predetermined movements relative to the work piece.

Preferably the robot is configured to receive data in the form of or derived from one or more images of the work piece and the markers and information concerning the predetermined movements, the predetermined movements being defined within a frame of reference relative to the markers.

Conveniently the robot is for use by a surgeon, the controllable arm being adapted to carry a surgeon's instrument or tool.

Advantageously the robot is in combination with an arrangement provided with elements to engage the work piece to connect the arrangement to the work piece, the arrangement carrying said indicator.

Conveniently the indicator is removably connected to said arrangement.

Preferably the indicator has a head defining a planar face, the face being marked to indicate an axis passing across the face, the head being carried by a stem, the stem being received in a socket on the arrangement.

According to another aspect of this invention there is provided a method of registering a work piece relative to a robot, the method comprising the steps of acquiring one or more images of a work piece, which incorporates visual markers, processing the images to identify at least one point on the work piece, generating control signals for a robot to define a path to be followed by a tool or instrument carried by the robot to bring the tool or instrument to said point, providing the robot with an image acquisition device, utilising the image acquisition device to acquire images of the markers, utilising a processor to process the images acquired with said image acquisition device and to control the robot to move the tool or instrument along said path, providing an indicator which has a predetermined spatial position relative to the markers, processing within the processor the images from the said image acquisition device to determine the position of the indicator, concealing the markers, monitoring the position of the indicator and responding to a movement of the indicator relative to the frame of reference of the robot by controlling the robot so that the tool or instrument continues to move along said path.

Conveniently the indicator is removably mounted on an arrangement which is secured to the work piece, the method comprising the steps of removing the indicator from the said arrangement prior to the concealing of the markers, and replacing the indicator with an identical, but sterile, indicator following the concealing of the markers.

Advantageously the concealing of the markers is effected by applying sterile drapes to the work piece.

Preferably the step of acquiring one or more images is effected utilising an X-ray or NMR or ultrasound apparatus.

Conveniently the step of processing the images is effected using a human operator to analyse the images, and to use a pointer to identify said at least one point on the work piece.

In order that the invention may be more readily understood, and so that further features thereof may be appreciated, the invention will now be described, by way of example, with reference to the accompanying drawings in which:
FIGURE 1 is a diagrammatic view of an apparatus for taking an image of a "work piece" in the form of the head of a patient,
FIGURE 2 is a block diagrammatic view,
FIGURE 3 is a view of a stereotactic frame provided with an indicator,

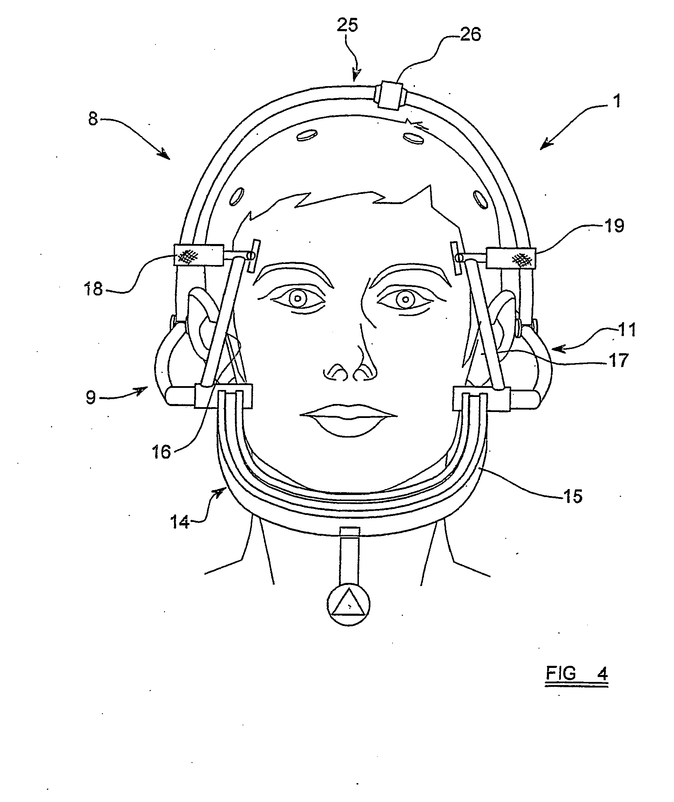

FIGURE 4 is a view of the stereotactic frame applied to the head of the patient,
FIGURE 5 is a view of the patient, with the stereotactic frame, with the stereotactic frame secured to an operating table, the figure also illustrating a robot,
FIGURE 6 is a view similar to that of Figure 5 showing an indicator mounted on the stereotactic frame,
FIGURE 7 is a view corresponding to Figure 6 showing the patient when draped, with the indicator protruding,
FIGURE 8 is a block diagram, and
FIGURE 9 is a further block diagram.

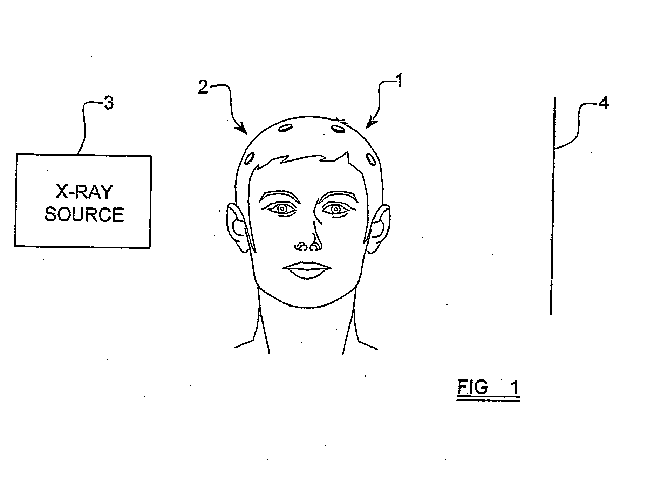

Referring initially to Figure 1 of the accompanying drawings, a work piece, to be operated on with the aid of a computer controlled robot, is illustrated in the form of a human head 1. Mounted on the head are a plurality of markers 2. The markers are visible markers and are mounted on the exterior of the head so as to be readily seen. The markers, in this embodiment, are radio-opaque.

The head or work piece 1 is illustrated in position in an image acquisition apparatus between an x-ray source 3 and an x-ray sensitive screen 4. An x-ray image of the head can thus be taken, with the image including, of course, the radio-opaque markers 2.

It is envisaged that a plurality of images will be taken, with the work piece or head in different positions relative to the x-ray source and the screen, and this will enable the resultant set of images to be processed to produce a three-dimensional recreation of the work piece together with the markers, or three orthogonal images. Of course, the image-taking apparatus may be a CAT (Computerised Axial Topography) apparatus, producing a series of images equivalent to successive cross-sectional views or "slices", and whilst the invention has been described thus far with reference to the taking of an x-ray image, it is to be appreciated that many other imaging techniques may be utilised, including NMR and ultrasound techniques.

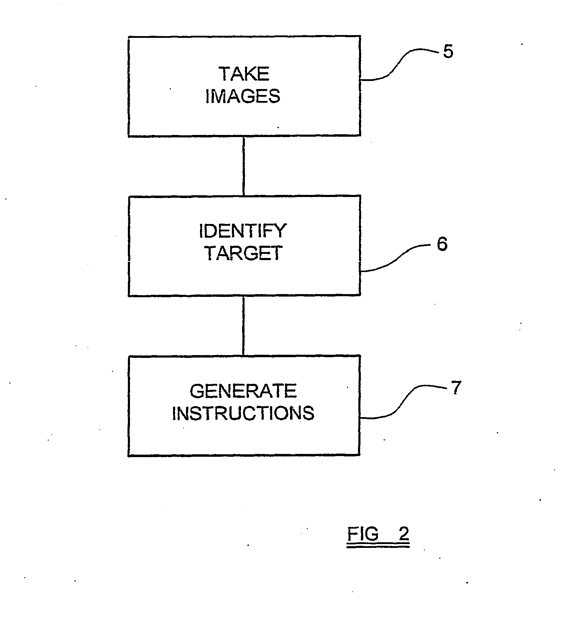

Referring now to Figure 2, after a plurality of images have been taken, 5, the images are processed to identify a target within the human head 1. The target may, for example, be a tumour. The identification of the target, stage 6 as shown in Figure 2, may be carried out by considering the plurality of images, and, optionally, by processing the images by computer. The target may be specifically identified, as described above, by a human operator using a pointer.

Subsequent to identification of the target, a series of instructions are generated 7 for a robot, the instructions indicating the desired path of travel of a tool or instrument carried by the robot. The instructions are generated to define predetermined movements of tool or instrument carried by the robot in three-dimensional space, that three-dimensional space being identified by a frame of reference or set of spatial co-ordinates, and the same frame of reference and set of spatial co-ordinates is used to determine the precise position of each of the markers 2. The instructions thus, effectively, determine a particular predetermined movement of a tool or instrument relative to the markers 2.

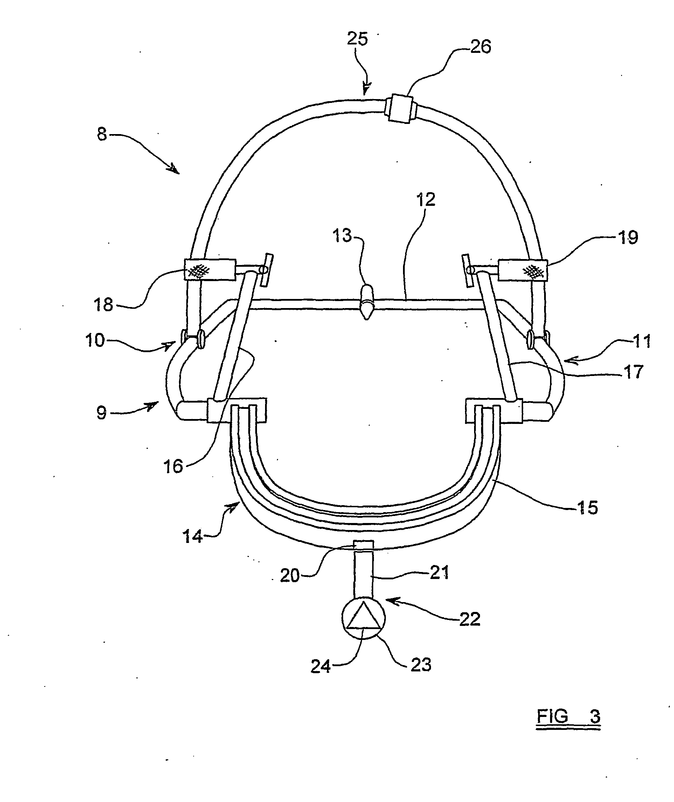

Subsequently the head of the patient is provided with a stereotactic frame. Figure 3 illustrates a typical stereotactic frame although it is to be understood that many models of stereotactic frame exist.

Referring to Figure 3, the illustrated stereotactic frame 8 is provided with a base ring 9 which is configured to be mounted over the head of the patient. The base ring 9 comprises two substantially horizontal side arms 10, 11 which are interconnected by a rear bar 12. The rear bar 12 carries a mounting screw 13. The forward ends of the side arms 10 and 11 are interconnected by a yoke 14, the yoke 14 having a forwardly protruding U-shaped section 15 to be located in front of the jaw of the patient. The yoke is provided, at either side of the U-shaped section 15, with an upstanding arm 16, 17, each upstanding arm carrying, at its upper end, a mounting screw 18, 19.

The U-shaped yoke is provided with a socket 20 to receive the stem 21 of an indicator 22. The indicator 22 comprises a stem 21 and a head 23, the head 23 being provided with a marking 24, on a planar face of the head, to indicate an axis passing across the planar face to show the precise orientation of the head. As will be understood, the precise design of the indicator is not critical to the invention, but the indicator does need to be designed in such a way that by analysing visual images of the indicator it is possible to determine the precise position and orientation of the indicator in three-dimensional space. In the described embodiment the indicator is removably connected to the stereotactic frame.

The illustrated stereotactic frame is provided with an arcuate half-hoop 25 which extends upwardly above the two side arms 10 and 11, the half-hoop 25 slideably supporting a tool carrier 26.

It is to be understood that when the indicator 22 is mounted in position on the stereotactic frame, the head 23 of the indicator has a precisely predetermined position, in three-dimensional space, relative to the rest of the stereotactic frame. Because the stereotactic frame is fitted relative to the head 1, and is thus fixed relative to the markers 2, the head 23 of the indicator 22 has a precisely determined spatial relationship with the markers 2. Thus it is possible, if the precise position and orientation of the head 23 of the indicator is known in a specific frame of reference, the position of the markers within that frame of reference can be easily determined even if the markers 2 are concealed.

It is to be appreciated that the stereotactic frame, as described above, is to be mounted on the head 1 of the patient, by placing the base ring over the head of the patient and subsequently tightening the mounting screws 13, 18 and 19 until they engage bony parts of the skull of the patient. The stereotactic frame is thus firmly mounted in position relative to the head of the patient.

The patient may then be placed on an operating table 30 and the stereotactic frame may be clamped to the operating table by an appropriate clamp 31.
The stereotactic frame is thus securely fixed in position.

At this stage the markers 2 are visible.

A robot 40 is provided. The robot 40 comprises a housing 41 which is fixed in position, and which is thus in a predetermined spatial relationship with the stereotactic frame 8 which is clamped to the operating table 30. The housing 41 carries a vertical supporting column 42, the upper end of which pivotally supports an intermediate arm 43 which, in its turn, carries, at its free end, a pivotally mounted tool or instrument carrying arm 44. Mounted on the tool or instrument carrying arm 44 is a camera 45. The tool or instrument carrying arm 44 is illustrated carrying a tool or instrument 46. Of course, many different types of robots can be envisaged for use with the invention.

The camera 45 may be any form of camera such as a television camera, a digital camera, a CCD device or the like. The camera is adapted to acquire visual images of the head 1 of the patient and the markers 2 and, as will be described below, is also adapted to acquire visual images of the indicator 22.

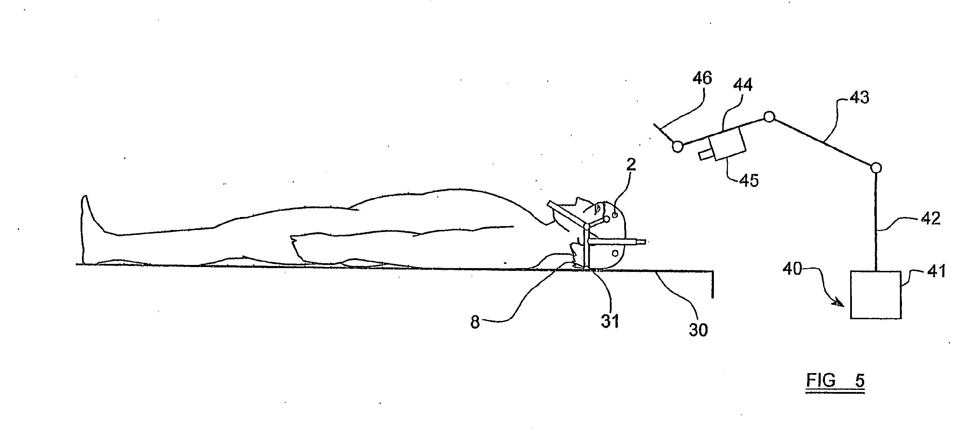

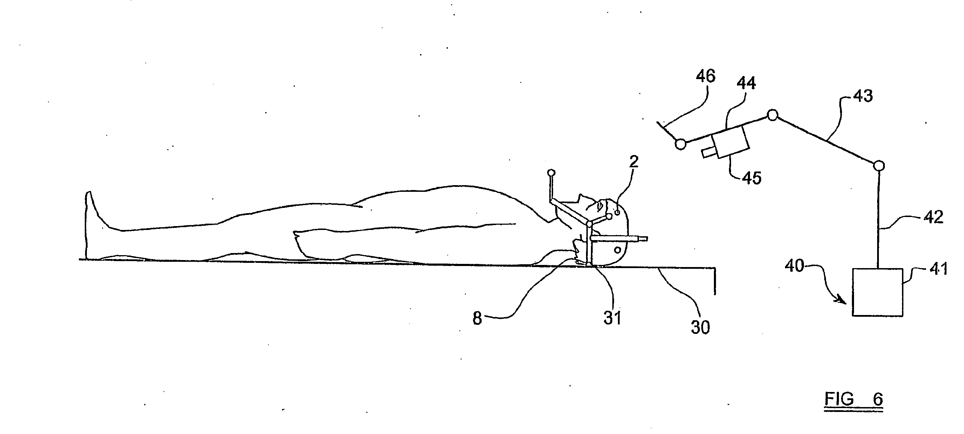

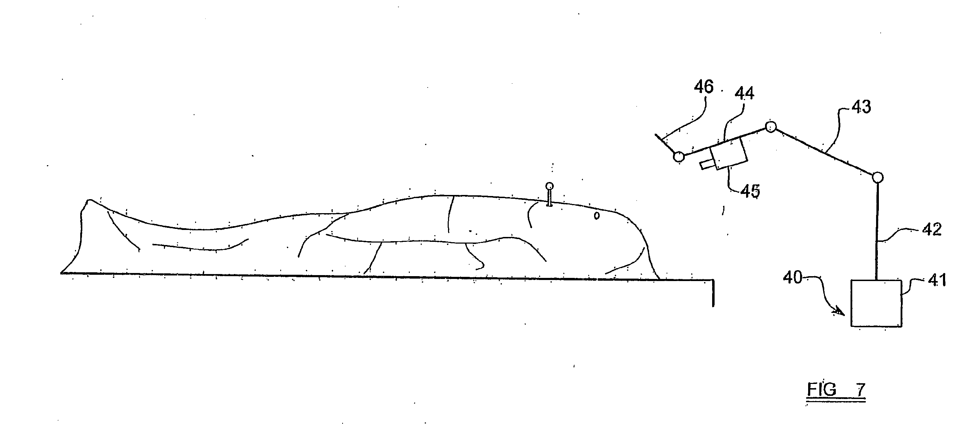

Figure 6 is a view corresponding to Figure 5 illustrating an indicator 22 mounted to the stereotactic frame. The head 23 of the indicator 22 has a predetermined spatial relationship with the head of the patient. Figure 7 illustrates the situation that exists when the patient has been covered with sterile drapes; thus concealing the markers 2 but leaving a sterile indicator exposed.

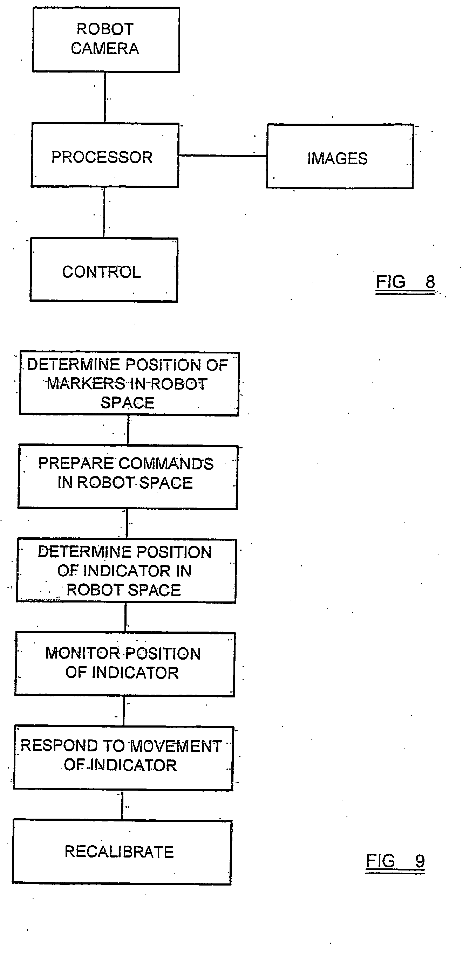

It is to be understood that when the patient is initially located on the operating table it is necessary to "register" the patient relative to the robot, so that the instructions that have been generated identifying the path to be followed by the tool or instrument carried by the robot can be "translated" into the frame of reference or spatial co-ordinates of the robot itself. Thus, initially, the camera 45 acquires images of the head 1, when the camera is in specific positions, and images from the camera are passed to a processor, as shown in Figure 8. The processor also receives images or data derived from the image acquisition apparatus of Figure 1, and the processor effectively correlates the frame of reference utilised in the image acquisition apparatus of Figure 1 with the frame of reference of the robot. The processor can thus pass signals to the control arrangement of the robot so that a tool or instrument carried by the instrument carrying arm 44 of the robot performs a desired manoeuvre relative to the patient.

Referring now to Figure 9 it is to be appreciated that the processor, on receiving images from the robot camera 45 effectively determines the position of the markers 2 in the frame of reference of the robot. Since the position of the markers is known, with regard to the frame of reference of the image acquiring apparatus, the processor can correlate the two frames of reference and can prepare appropriate commands in the robot's internal frame of reference.

The processor subsequently determines the position of the indicator 22, when the indicator has been mounted on the stereotactile frame, with regard to the internal frame of reference of the robot. Since the indicator 22 has a predetermined spatial relationship to the stereotactile frame, and thus also to the markers 2, the processor can determine the absolute spatial relationship between the indicator 22 and the frame of reference of the patient as utilised by the image acquisition device of Figure 1.

Therefore, when the patient is covered (see Figure 7) and the markers 2 are concealed from the view of the robot camera 45, the robot camera 45 can capture images of the indicator 22 (which is visible to the robot camera 45) and these images allow the processor to determine the position of the markers 2 in the frame of reference of the robot.

It will be appreciated that the processor is configured to determine the position of the indicator 22 and to respond to movement of the indicator 22 within the spatial frame of reference of the robot when the markers 2 are concealed to determine the new position of the indicator 22 and thus the new position of the patient. Subsequently, the processor can control the robot to continue effecting a predetermined movement relative to the patient based upon the new position of the patient.

It is to be appreciated that when the initial image of the indicator 22 is acquired, the indicator 22 may be a sterile indicator, appropriately mounted on the stereotactile frame before the patient is draped. The indicator may remain in place as the patient is draped leaving the sterile indicator protruding above the drapes which cover the patient.

Alternatively, however, the indicator that is utilised during the procedure illustrated in Figure 6 may be a non-sterile indicator, and this may be removed, prior to draping to be replaced by a sterile indicator inserted through an appropriate opening in the sterile drapes, after the sterile drapes have been located in place. If this expedient is utilised it is essential that the socket 20 in the stereotactile frame which receives the stem 21 of the indicator 22 should be such that the stem 21 of the indicator 22 can only be placed in the socket 20 in one particular orientation and with one particular degree of insertion. In such a way a non-sterile indicator may be used for the acquisition of the image of the indicator by the camera 45 provided on the robot, and this indicator may be replaced by an absolutely identical, but sterile, indicator after the draping procedures have been completed. The head of the sterile indicator will then occupy exactly the same position, relative to the stereotactile frame, as the head of the non-sterile indicator.

The camera 45 will, as an operation is performed on the patient, continue to acquire images of the indicator. The processor is programmed to determine the position of the indicator within the frame of reference of the computer at regular intervals, and to determine if the indicator has moved. If the indicator has moved, as a consequence of an undesired movement of the head of the patient (or even as a consequence of a desired and required movement of the head of the patient), the processor, on receiving images of the indicator in its new position from the camera 45, is programmed to determine the absolute position of the indicator within the frame of reference of the robot when the markers 2 are concealed and, because the absolute spatial relationship between the indicator and the markers present on the head of the patient is known, the processor can effectively recalibrate the robot, translating any instructions prepared on the frame of reference of the initial image acquisition device as shown in Figure 1, into appropriate instructions, within the frame of reference of the robot, having regard to the current position of the head of the patient.

Whilst the invention has been described with reference primarily to a work piece in the form of the head of a patient, it is to be appreciated that the invention may equally be used in connection with operations to be preformed on other parts of a patient, such as a knee or elbow or may be used on "work pieces" which are not part of a patient. Whilst the invention has been described with reference to a specific form of stereotactile frame, any appropriate form of retaining frame or clamp could be utilised.

The invention may be used during a surgical operation on a patient and the instrument 46 carried by the robot may be a surgical instrument.

When used in this Specification and Claims, the terms "comprises" and "comprising" and variations thereof mean that the specified features, steps or integers are included. The terms are not to be interpreted to exclude the presence of other features, steps or components.

## Claims

1. A robot (40), the robot being provided with a controllable arm (43,44) to carry an instrument or tool (46), **characterized in that** the robot (40) is provided with a visual image acquisition device (45) to obtain visual images of a work piece (1), including images of markers (2) and an indicator (22) present on the work piece (1), and **in that** the robot (40) incorporates a processor to process the images, the processor being configured to determine the position of the markers (2) within a spatial frame of reference of the robot (40) to determine the position of the work piece (1) in the spatial frame of reference of the robot (40) and to control the robot (40) to effect predetermined movements of an instrument or tool (46) carried by the arm (43,44) relative to the work piece (1), the processor being further configured to determine the position of said indicator (22) and to respond to movement of the said indicator (22) within the spatial frame of reference of the robot (40) when the markers (2) are concealed to determine the new position of the indicator (22) and thus the new position of the work piece (1) and subsequently to control the robot (40) to continue effecting the predetermined movements relative to the work piece (1).

2. A robot according to Claim 1 wherein the robot (40) is configured to receive data in the form of or derived from one or more images of the work piece (1) and the markers (2) and information concerning the predetermined movements, the predetermined movements being defined within a frame of reference relative to the markers (2).

3. A robot according to Claim 1 or Claim 2 for use by a surgeon, the controllable arm (43,44) being adapted to carry a surgeon's instrument or too (46).

4. A robot according to Claim 1 in combination with an arrangement (8) provided with elements (18,19) to engage the work piece (1) to connect the arrangement to the work piece, the arrangement carrying said indicator (22).

5. A robot (40) according to Claim 4 wherein the indicator (22) is removably connected to said arrangement (8).

6. A robot according to Claim 5 wherein the indicator (22) has a head (23) defining a planar face, the face being marked to indicate an axis passing across the face, the head (23) being carried by a stem (21), the stem (21) being received in a socket (20) on the arrangement (8).

7. A method of registering a work piece (1) relative to a robot (40), the method comprising the steps of acquiring one or more images of a work piece (1), which incorporates visual markers (2), processing the images to identify at least one point on the work piece (1), generating control signals for a robot (40) to define a path to be followed by a tool or instrument (46) carried by the robot (40) to bring the tool or instrument (46) to said point, **characterised in that** it Further comprises the step of providing the robot (40) with an image acquisition device (45), utilising the image acquisition device (45) to acquire images of the markers (2), utilising a processor to process the images acquired with said acquisition device (45) and to control the robot (40) to move the tool or instrument (46) along said path, providing an indicator (22) which has a predetermined spatial position relative to the markers (2), processing within the processor the images from the said image acquisition device (45) to determine the position of the indicator (22), concealing the markers (2), monitoring the position of the indicator (22) and responding to a movement of the indicator (22) relative to the frame of reference of the robot (40) by controlling the robot (40) so that the tool or instrument (46) continues to move along said path.

8. A method of registering a work piece (1) according to Claim 7 wherein the indicator (22) is removably mounted on an arrangement (8) which is secured to the work piece (1), the method comprising the steps of removing the indicator (22) from the said arrangement (8) prior to the concealing of the markers (2), and replacing the indicator (22) with an identical, but sterile, indicator following the concealing of the markers (2).

9. A method according to Claim 7 or Claim 8 wherein the concealing of the markers (2) is effected by applying sterile drapes to the work piece (1).

10. A method of registering a work piece (1) according to any one of Claims 7 to 9 wherein the step of acquiring one or more images is effected utilising an X-ray or NMR or ultrasound apparatus.

11. A method of registering a work piece (1) according to any one of Claims 7 to 10 wherein the step of processing the images is effected using a human operator to analyse the images, and to use a pointer to identify said at least one point on the work piece (1).

## Patentansprüche

1. Roboter (40), wobei der Roboter mit einem steuerbaren Arm (43, 44) versehen ist, um ein Instrument oder Werkzeug (46) zu tragen, **dadurch gekennzeichnet, dass** der Roboter (40) mit einem optischen Bilderfassungsgerät (45) zur Gewinnung optischer Bilder von einem Werkstück (1) versehen ist, wozu Bilder von Markierungen (2) und von einem auf dem Werkstück (1) vorhandenen Indikator (22) zählen, und **dadurch**, dass der Roboter (40) einen Prozessor zur Verarbeitung der Bilder enthält, wobei der Prozessor dafür konfiguriert ist, die Position der Markierungen (2) innerhalb eines räumlichen Bezugssystems des Roboters (40) zu ermitteln, um die Position des Werkstücks (1) in dem räumlichen Bezugssystem des Roboters (40) zu ermitteln und um den Roboter (40) so zu steuern, dass er vorgegebene Bewegungen eines von dem Arm (43, 44) getragenen Instruments oder Werkzeugs (46) im Verhältnis zum Werkstück (1) ausführt, wobei der Prozessor des Weiteren dafür konfiguriert ist, die Position des Indikators (22) zu ermitteln und auf Bewegung des Indikators (22) innerhalb des räumlichen Bezugssystems des Roboters (40) zu reagieren, wenn die Markierungen (2) verdeckt sind, um die neue Position des Indikators (22) zu ermitteln und damit die neue Position des Werkstücks (1) und um anschließend den Roboter (40) so zu steuern, dass dieser die Ausführung der vorgegebenen Bewegungen im Verhältnis zum Werkstück (1) fortsetzt.

2. Roboter gemäß Anspruch 1, wobei der Roboter (40) dafür konfiguriert ist, Daten in Form von oder abgeleitet aus einem oder mehreren Bildern des Werkstücks (1) und der Markierungen (2) sowie Informationen in Bezug auf die vorgegebenen Bewegungen zu empfangen, wobei die vorgegebenen Bewegungen innerhalb eines Bezugssystems im Verhältnis zu den Markierungen (2) definiert sind.

3. Roboter gemäß Anspruch 1 oder Anspruch 2 zur Verwendung durch einen Chirurgen, wobei der steuerbare Arm (43, 44) dazu angepasst ist, ein chirurgisches Instrument oder Werkzeug (46) zu tragen.

4. Roboter gemäß Anspruch 1 in Kombination mit einer Anordnung (8), die mit Elementen (18, 19) zum Eingreifen in das Werkstück (1) versehen ist, um die Anordnung mit dem Werkstück zu verbinden, wobei die Anordnung den Indikator (22) trägt.

5. Roboter (40) gemäß Anspruch 4, wobei der Indikator (22) entfernbar mit der Anordnung (8) verbunden ist.

6. Roboter gemäß Anspruch 5, wobei der Indikator (22) einen Kopf (23) hat, der eine planare Stirnfläche definiert, wobei die Stirnfläche so markiert ist, dass sie eine Achse bezeichnet, die über die Stirnfläche verläuft, wobei der Kopf (23) von einem Stamm (21) getragen wird und der Stamm (21) in einem Sockel (20) der Anordnung (8) aufgenommen wird.

7. Verfahren zum Registrieren eines Werkstücks (1) im Verhältnis zu einem Roboter (40), das Verfahren umfassend die Schritte des Erfassens von einem oder mehreren Bildern eines Werkstücks (1), welches optische Markierungen (2) enthält, des Verarbeitens der Bilder zur Identifizierung von mindestens einer Stelle des Werkstücks (1), des Erzeugens von Steuersignalen für einen Roboter (40), um einen Weg zu definieren, dem ein von dem Roboter (40) getragenes Werkzeug oder Instrument (46) folgen soll, um das Werkzeug oder Instrument (46) an die Stelle zu bringen, **dadurch gekennzeichnet, dass** das Verfahren des Weiteren die folgenden Schritte umfasst: das Versehen des Roboters (40) mit einem Bilderfassungsgerät (45), das Verwenden des Bilderfassungsgeräts (45) zum Erfassen von Bildern der Markierungen (2), das Verwenden eines Prozessors zum Verarbeiten der mit dem Bilderfassungsgerät (45) erfassten Bilder und zum Steuern des Roboters (40), so dass dieser das Werkzeug oder Instrument (46) entlang dem Weg bewegt, das Bereitstellen eines Indikators (22), der eine vorgegebene räumliche Position im Verhältnis zu den Markierungen (2) hat, das Verarbeiten der Bilder von dem Bilderfassungsgerät (45) in dem Prozessor zur Ermittlung der Position des Indikators (22), das Verdecken der Markierungen (2), das Überwachen der Position des Indikators (22) und das Reagieren auf eine Bewegung des Indikators (22) im Verhältnis zu dem Bezugssystem des Roboters (40), indem der Roboter (40) so gesteuert wird, dass das Werkzeug oder Instrument (46) die Bewegung entlang des Weges fortsetzt.

8. Verfahren zum Registrieren eines Werkstücks (1) gemäß Anspruch 7, wobei der Indikator (22) entfernbar an einer Anordnung (8) montiert ist, die an dem Werkstück (1) befestigt ist, das Verfahren umfassend die Schritte des Entfernens des Indikators (22) von der Anordnung (8) vor dem Verdecken der Markierungen (2) und das Ersetzen des Indikators (22) durch einen identischen, jedoch sterilen Indikator im Anschluss an das Verdecken der Markierungen (2).

9. Verfahren gemäß Anspruch 7 oder Anspruch 8, wobei das Verdecken der Markierungen (2) durch Anbringung steriler Abdecktücher an dem Werkstück (1) bewirkt wird.

10. Verfahren zum Registrieren eines Werkstücks (1) gemäß einem der Ansprüche 7 bis 9, wobei der Schritt des Erfassens von einem oder mehreren Bildern unter Verwendung einer Röntgen- oder NMR- oder Ultraschalleinrichtung bewirkt wird.

11. Verfahren zum Registrieren eines Werkstücks (1) gemäß einem der Ansprüche 7 bis 10, wobei der Schritt des Verarbeitens der Bilder unter Einsatz einer menschlichen Bedienkraft bewirkt wird, um die Bilder zu analysieren und um einen Zeiger zum Identifizieren der mindestens einen Stelle des Werkstücks (1) zu verwenden.

## Revendications

1. Un robot (40), le robot étant muni d'un bras contrôlable (43, 44) pour porter un instrument ou un outil (46), **caractérisé en ce que** le robot (40) est muni d'un dispositif d'acquisition d'images visuelles (45) pour obtenir des images visuelles d'une pièce à travailler (1), y compris des images de marqueurs (2) et d'un indicateur (22) présents sur la pièce à travailler (1), et **en ce que** le robot (40) incorpore un processeur pour traiter les images, le processeur étant configuré pour déterminer la position des marqueurs (2) à l'intérieur d'un système de référence spatial du robot (40) pour déterminer la position de la pièce à travailler (1) dans le système de référence spatial du robot (40) et pour commander le robot (40) pour effectuer des déplacements prédéterminés d'un instrument ou d'un outil (46) porté par le bras (43, 44) relativement à la pièce à travailler (1), le processeur étant de plus configuré pour déterminer la position dudit indicateur (22) et pour répondre au déplacement dudit indicateur (22) à l'intérieur du système de référence spatial du robot (40) lorsque les marqueurs (2) sont dissimulés pour déterminer la nouvelle position de l'indicateur (22) et de ce fait la nouvelle position de la pièce à travailler (1) et ensuite pour commander le robot (40) afin de continuer à effectuer les déplacements prédéterminés relativement à la pièce à travailler (1).

2. Un robot selon la revendication 1, dans lequel le robot (40) est configuré pour recevoir des données sous la forme ou dérivées d'une ou de plusieurs image(s) de la pièce à travailler (1) et des marqueurs (2) et des informations concernant les déplacements prédéterminés, les déplacements prédéterminés étant définis à l'intérieur d'un système de référence relativement aux marqueurs (2).

3. Un robot selon la revendication 1 ou la revendication 2, destiné à être utilisé par un chirurgien, le bras contrôlable (43, 44) étant adapté pour porter un instrument ou un outil d'un chirurgien (46).

4. Un robot selon la revendication 1 en combinaison avec un agencement (8) muni d'éléments (18, 19) pour se mettre en prise avec la pièce à travailler (1) afin de raccorder l'agencement à la pièce à travailler, l'agencement portant ledit indicateur (22).

5. Un robot (40) selon la revendication 4, dans lequel l'indicateur (22) est raccordé de façon amovible audit agencement (8).

6. Un robot selon la revendication 5, dans lequel l'indicateur (22) a une tête (23) définissant une face planaire, la face étant marquée pour indiquer un axe passant à travers la face, la tête (23) étant portée par une tige (21), la tige (21) étant reçue dans une douille (20) sur l'agencement (8).

7. Un procédé pour enregistrer une pièce à travailler (1) relativement à un robot (40), le procédé comportant les étapes consistant à acquérir une ou plusieurs image(s) d'une pièce à travailler (1), qui incorpore des marqueurs visuels (2), traiter les images pour identifier au moins un point sur la pièce à travailler (1), générer des signaux de commande pour un robot (40) pour définir un chemin devant être suivi par un outil ou un instrument (46) porté par le robot (40) pour amener l'outil ou l'instrument (46) audit point, **caractérisé en ce qu'**il comporte les étapes supplémentaires consistant à munir le robot (40) d'un dispositif d'acquisition d'images (45), utiliser le dispositif d'acquisition d'images (45) pour acquérir des images des marqueurs (2), utiliser un processeur pour traiter les images acquises avec ledit dispositif d'acquisition (45) et commander le robot (40) pour déplacer l'outil ou l'instrument (46) suivant ledit chemin, fournir un indicateur (22) qui a une position spatiale prédéterminée relativement aux marqueurs (2), traiter à l'intérieur du processeur les images dudit dispositif d'acquisition d'images (45) pour déterminer la position de l'indicateur (22), dissimuler les marqueurs (2), surveiller la position de l'indicateur (22) et répondre à un déplacement de l'indicateur (22) relativement au système de référence du robot (40) en commandant le robot (40) de façon à ce que l'outil ou l'instrument (46) continue à se déplacer suivant ledit chemin.

8. Un procédé pour enregistrer une pièce à travailler (1) selon la revendication 7, dans lequel l'indicateur (22) est monté de façon amovible sur un agencement (8) qui est assujetti à la pièce à travailler (1), le procédé comportant les étapes consistant à retirer l'indicateur (22) dudit agencement (8) avant la dissimulation des marqueurs (2), et remplacer l'indicateur (22) par un indicateur identique, mais stérile, après la dissimulation des marqueurs (2).

9. Un procédé selon la revendication 7 ou la revendication 8, dans lequel la dissimulation des marqueurs (2) est effectuée en appliquant des champs stériles sur la pièce à travailler (1).

10. Un procédé pour enregistrer une pièce à travailler (1) selon n'importe laquelle des revendications 7 à 9, dans lequel l'étape consistant à acquérir une ou plusieurs image(s) est effectuée à l'aide d'un appareil à rayons X ou à résonance magnétique nucléaire ou à ultrasons.

11. Un procédé pour enregistrer une pièce à travailler (1) selon n'importe laquelle des revendications 7 à 10, dans lequel l'étape consistant à traiter les images est effectuée à l'aide d'un opérateur humain pour analyser les images, et pour utiliser un pointeur afin d'identifier ledit au moins un point sur la pièce à travailler (1).
